(19) Europäisches Patentamt
European Patent Office
Office européen des brevets

(11) EP 0 698 593 A1

# (12) DEMANDE DE BREVET EUROPEEN

(43) Date de publication:
28.02.1996 Bulletin 1996/09

(51) Int Cl.[6]: **C07C 45/46**, C07C 49/84, C07C 49/825

(21) Numéro de dépôt: **95401929.5**

(22) Date de dépôt: **22.08.1995**

(84) Etats contractants désignés:
**AT BE CH DE DK ES FR GB IE IT LI NL**

(30) Priorité: **24.08.1994 FR 9410253**

(71) Demandeur: **RHONE-POULENC CHIMIE**
**F-92408 Courbevoie Cédex (FR)**

(72) Inventeurs:
- **Dubac, Jacques**
  **F-31320 Pechbusque (FR)**
- **Labrouillere, Mireille**
  **F-47000 Agen (FR)**
- **Laporterie, André**
  **F-31450 Pompertuzat (FR)**
- **Desmurs, Jean-Roger**
  **F-69360 St. Symphorien d'Ozon (FR)**

(74) Mandataire: **Dutruc-Rosset, Marie-Claude et al**
**F-92408 Courbevoie Cédex (FR)**

## (54) Procédé d'acylation d'un compose aromatique

(57) La présente invention a pour objet un procédé d'acylation d'un composé aromatique porteur d'au moins un groupe hydroxyle et/ou alkoxy. Plus particulièrement, l'invention a trait à un procédé d'acylation d'un éther aromatique, en para du groupe alkoxy porté par le noyau aromatique.

Dans sa variante préférée, l'invention réside dans un procédé de condensation du chlorure d'acétyle ou de l'anhydride acétique avec un éther aromatique.

L'invention s'applique à la préparation d'alkylcétones alkoxyaromatiques.

Le procédé d'acylation d'un composé aromatique porteur d'au moins un groupe hydroxyle et/ou alkoxy selon l'invention qui consiste à faire réagir ledit composé aromatique avec un agent d'acylation, en présence d'un catalyseur puis à récupérer le produit formé est caractérisé par le fait que l'on conduit la réaction d'acylation en présence d'une quantité efficace d'un halogénure de bismuth ou tout composé susceptible de le générer in situ.

EP 0 698 593 A1

## Description

La présente invention a pour objet un procédé d'acylation d'un composé aromatique porteur d'au moins un groupe hydroxyle et/ou alkoxy. Plus particulièrement, l'invention a trait à un procédé d'acylation d'un éther aromatique, en para du groupe alkoxy porté par le noyau aromatique.

Dans sa variante préférée, l'invention réside dans un procédé de condensation du chlorure d'acétyle ou de l'anhydride acétique avec un éther aromatique.

L'invention s'applique à la préparation d'alkylcétones alkoxyaromatiques.

Un procédé classique de préparation de cétones aromatiques consiste à effectuer une réaction d'acylation de type Friedel-Crafts.

On fait réagir le composé aromatique et un agent d'acylation, en présence d'un catalyseur qui est généralement le chlorure d'aluminium.

Une illustration de ce type de procédé est donné par les travaux de C. KURODA et al [Sci. Papers Inst. Phys. Chem. Res. 18, pp. 51-60 (1932)] qui ont décrit la préparation de méthoxyacétophénones, par réaction d'un composé aromatique porteur de 1 à 3 groupes méthoxy, avec du chlorure d'acétyle, en présence de chlorure d'aluminium.

Toutefois, la mise en oeuvre du chlorure d'aluminium présente de nombreux inconvénients. Le chlorure d'aluminium est un produit corrosif et irritant. De plus, il est nécessaire de mettre en oeuvre une quantité importante de chlorure d'aluminium au moins égale à la stoechiométrie, par suite de la complexation de la cétone formée. En conséquence, le chlorure d'aluminium n'est donc pas un vrai catalyseur.

En fin de réaction, il est nécessaire d'éliminer le chlorure d'aluminium du milieu réactionnel en faisant une hydrolyse acide ou basique.

Cette technique d'hydrolyse implique l'addition d'eau dans le milieu réactionnel ce qui complique notablement la mise en oeuvre du procédé car le cation métallique, et plus particulièrement le cation aluminium forme alors en présence d'eau, des complexes polyoxo- et/ou polyhydroxo- d'aluminium de consistance laiteuse, difficiles ensuite à séparer. Il en résulte la nécessité de faire un traitement long et coûteux comportant après l'hydrolyse, une extraction de la phase organique, une séparation des phases aqueuse et organique, voire-même un séchage de cette dernière. La séparation du chlorure d'aluminium est donc longue et coûteuse.

Par ailleurs, se pose le problème des effluents aqueux salins qu'il faut ensuite neutraliser ce qui impose une opération supplémentaire.

De plus, le chlorure d'aluminium ne peut être recyclé du fait de son hydrolyse.

Pour pallier cet inconvénient, Atsushi KAWADA et al [J. Chem. Soc. Chem. Commun. pp. 1158 (1993)] ont proposé d'effectuer la réaction d'acyclation d'un composé aromatique par l'anhydride acétique en présence d'une quantité catalytique de trifluorométhanesulfonate de lanthanide, et en particulier d'ytterbium. Cependant, le catalyseur utilisé est un produit coûteux et difficile à synthétiser.

L'objet de la présente invention est de fournir un procédé permettant d'obvier aux inconvénients précités.

Il a maintenant été trouvé et c'est ce qui constitue l'objet de la présente invention un procédé d'acylation d'un composé aromatique porteur d'au moins un groupe hydroxyle et/ou alkoxy qui consiste à faire réagir ledit composé aromatique avec un agent d'acylation, en présence d'un catalyseur, puis à récupérer le produit formé caractérisé par le fait que l'on conduit la réaction d'acylation en présence d'une quantité efficace d'un halogénure de bismuth ou tout composé susceptible de le générer in situ.

Dans l'exposé qui suit de la présente invention, on entend "par composé aromatique", un composé aromatique dont un atome d'hydrogène directement lié au noyau aromatique est remplacé par un groupe hydroxyle et/ou un groupe alkoxy tel que défini ci-après, et par "aromatique", la notion classique d'aromaticité telle que définie dans la littérature, notamment par Jerry MARCH, Advanced Organic Chemistry, 4ème édition, John Wiley and Sons, 1992, pp. 40 et suivantes.

Le procédé de l'invention s'applique aux composés aromatiques qui sont plus particulièrement des composés phénoliques et/ou des éthers aromatiques.

Plus précisément, la présente invention a pour objet un procédé d'acylation d'un composé aromatique porteur d'au moins un groupe hydroxyle et/ou alkoxy de formule générale (I) :

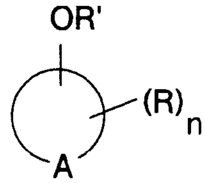

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,

- R représente un ou plusieurs substituants, identiques ou différents,

- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,

- n est un nombre inférieur ou égal à 4.

Dans le présent texte, on désigne, de manière simplifiée, par "groupes alkoxy", les groupes du type R'-O- dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant cyclique.

Le composé aromatique qui intervient dans le procédé de l'invention répond à la formule (I) dans laquelle R' représente un radical aliphatique acyclique, saturé ou insaturé, linéaire ou ramifié.

Plus préférentiellement, R' représente un radical alkyle linéaire ou ramifié ayant de 1 à 12 atomes de carbone, de préférence de 1 à 6 atomes de carbone,: la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène).

Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.

Le radical aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux composés aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle, éthyle, propyle, isopropyle, butyle, isobutyle, sec-butyle, tert-butyle ou phényle.

Dans la formule générale (I) des composés aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés.

Le reste A peut porter un ou plusieurs substituants sur le noyau aromatique.

Des exemples de substituants R sont donnés ci-après mais cette liste ne présente pas de caractère limitatif. N'importe quel substituant peut être présent sur le cycle dans la mesure où il n'interfère pas au niveau du produit désiré.

Le reste A pouvant entre autres porter plusieurs groupes alkoxy, il est possible selon le procédé de l'invention d'acyler des composés polyalkoxylés.

Le procédé de l'invention s'applique plus particulièrement, aux composés aromatiques de formule (Ia) :

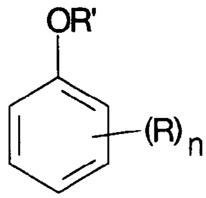

(Ia)

dans laquelle :

- n est un nombre inférieur ou égal à 4, de préférence égal à 0, 1 ou 2,

- le radical R' représente un atome d'hydrogène ou un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,

- le ou les radicaux R représentent l'un des atomes ou groupes suivants :

  . un atome d'hydrogène,

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,

  . un groupe hydroxyle,

  . un atome d'halogène, de préférence un atome de fluor, chlore ou brome.

- les radicaux R' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux R' et R peuvent représenter un radical méthylène dioxy ou éthylène dioxy.

Le procédé de l'invention s'applique plus particulièrement aux éthers aromatiques de formule (Ia) dans laquelle n est égal à 1, les radicaux R et R' représentant tous deux, des radicaux alkoxy, identiques ou différents.

A titre illustratif de composés répondant à la formule (I), on peut mentionner plus particulièrement :

- des composés phénoliques tels que le phénol, l'o-crésol, le gaïacol,

- des monoéthers tels que l'anisole, l'éthoxybenzène (phénétole), le butoxybenzène, l'isobutoxybenzène, le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-tert-butylanisole, le 3-tert-butylanisole, le 2-benzylanisole, le 2-cyclohexylanisole, le 1-bromo 2-éthoxybenzène, le 1-bromo 3-éthoxybenzène, le 1-chloro 2-éthoxybenzène, le 1-chloro 3-éthoxybenzène, le 1-éthoxy 2-éthylbenzène, le 1-éthoxy 3-éthylbenzène, le 2,3-diméthylanisole, le 2,5-diméthylanisole,

- des diéthers comme le vératrole, le 1 ,3-diméthoxybenzène, le 1,2-diéthoxybenzène, le 1,3-diéthoxybenzène, le 1 ,2-dipropoxybenzène, le 1,3-dipropoxybenzène, le 1,2-méthylènedioxybenzène, le 1,2-éthylènedioxybenzène,

- des triéthers comme le 1,2,3-triméthoxybenzène, le 1 ,3,5-triméthoxybenzène, le 1 ,3,5-triéthoxybenzène.

Les composés auxquels s'applique de manière plus particulièrement intéressante le procédé selon l'invention sont

le phénol, l'anisole et le vératrole.

Pour ce qui est du réactif d'acylation, il répond plus particulièrement à la formule (II) :

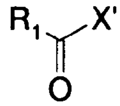

(II)

dans laquelle :

- $R_1$ représente :
  . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 8 atomes de carbone ; un radical aliphatique saturé ou insaturé, linéaire ou ramifié, porteur d'un substituant cyclique,

- X' représente :

  . un atome d'halogène, de préférence un atome de chlore ou de brome,

  . un radical -O-CO-$R_2$ avec $R_2$, identique ou différent de $R_1$, ayant la même signification que $R_1$,

Par substituant cyclique, on se réfère à ce qui est décrit précédemment.

Plus préférentiellement, $R_1$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène ou un groupe $CF_3$).

$R_1$ représente de préférence un radical alkyle ayant de 1 à 4 atomes de carbone, tel que méthyle, éthyle, propyle, isopropyle, butyle, isobutyle, sec-butyle, tert-butyle.

Le radical $R_1$ représente également d'une manière préférentielle un radical phényle qui peut être éventuellement substitué. N'importe quel substituant peut être présent sur le cycle dans la mesure où il n'interfère pas au niveau du produit souhaité.

Comme exemples plus particuliers de substituants, on peut citer, notamment:

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,

. un groupe hydroxyle,

. un atome d'halogène, de préférence un atome de fluor, chlore ou brome.

Les agents d'acylation préférés répondent à la formule (II) dans laquelle X représente un atome de chlore et $R_1$ représente un radical méthyle ou éthyle.

Lorsque l'agent d'acylation est un anhydride d'acide, les composés préférés répondent à la formule (II) dans laquelle $R_1$ et $R_2$ sont identiques et représentent un radical alkyle ayant de 1 à 4 atomes de carbone.

A titre illustratif d'agents d'acylation répondant à la formule (II), on peut citer plus particulièrement:

- le chlorure d'acétyle,

- le chlorure de propanoyle,

- le chlorure d'isobutanoyle,

- le chlorure de pivaloyle,

- l'anhydride acétique,

- l'anhydride isobutyrique,
- l'anhydride trifluoroacétique.

Conformément au procédé de l'invention, on effectue la réaction d'acylation d'un composé aromatique porteur d'au moins un groupe hydroxyle et/ou alkoxy en présence d'un catalyseur qui est choisi parmi les halogénures de bismuth ou tout composé susceptible de le générer in situ.

Les composés préférés sont les halogénures de bismuth de préférence, chlorure, bromure, iodure et plus préférentiellement le chlorure de bismuth $BiCl_3$; le bromure de bismuth $BiBr_3$; l'iodure de bismuth $BiI_3$.

On peut également mettre en oeuvre tout composé du bismuth dans la mesure où il est associé à une source d'halogène.

Ainsi, on peut faire appel au bismuth métallique ou à tout dérivé minéral ou organique du bismuth. Le reste associé au bismuth n'est pas critique.

Des composés illustratifs de catalyseurs qui peuvent être utilisés dans le procédé selon la présente invention sont : les oxydes de bismuth ; les hydroxydes de bismuth ; les sels d'hydracides minéraux tels que : sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth, oxyde de bismuth et de germanium.

D'autres composés appropriés sont également des sels d'acides organiques aliphatiques ou aromatiques tels que: acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropolyacides contenant du bismuth ainsi que leurs sels ; conviennent également les bismuthines aliphatiques et aromatiques.

A titre d'exemples spécifiques, on peut citer:

- comme oxydes: BiO ; $Bi_2O_3$ : $Bi_2O_4$ ; $Bi_2O_5$,
- comme hydroxydes : $Bi(OH)_3$,
- comme sels d'hydracides minéraux : le sulfure de bismuth $Bi_2S_3$ ; le séléniure de bismuth $Bi_2Se_3$ : le tellurure de bismuth $Bi_2Te_3$,
- comme sels d'oxyacides minéraux : le sulfite basique de bismuth $Bi_2(SO_3)_3$, $Bi_2O_3$, $5H_2O$ ; le sulfate neutre de bismuth $Bi_2(SO_4)_3$ ; le sulfate de bismuthyle $(BiO)HSO_4$ ; le nitrite de bismuthyle $(BiO)NO_2$, $0,5H_2O$ ; le nitrate neutre de bismuth $Bi(NO_3)_3$, $5H_2O$ ; le nitrate double de bismuth et de magnésium $2Bi(NO_3)_3$, $3Mg(NO_3)_2$, $24H_2O$ ; le nitrate de bismuthyle $(BiO)NO_3$ ; le phosphite de bismuth $Bi_2(PO_3H)_3$, $3H_2O$ ; le phosphate neutre de bismuth $BiPO_4$ ; le pyrophosphate de bismuth $Bi_4(P_2O_7)_3$ ; le carbonate de bismuthyle $(BiO)_2CO_3$, $0,5H_2O$ ; le perchlorate neutre de bismuth $Bi(ClO_4)_3$, $5H_2O$ ; le perchlorate de bismuthyle $(BiO)ClO_4$ ; l'antimoniate de bismuth $BiSbO_4$ ; l'arséniate neutre de bismuth $Bi(AsO_4)_3$ ; l'arséniate de bismuthyle $(BiO)AsO_4$, $5H_2O$ ; le sélénite de bismuth $Bi_2(SeO_3)_3$,
- comme sels d'oxyacides dérivés de métaux de transition : le vanadate de bismuth $BiVO_4$ ; le niobate de bismuth $BiNbO_4$ : le tantalate de bismuth $BiTaO_4$ ; le chromate neutre de bismuth $Bi_2(CrO_4)_3$, $3,5H_2O$ ; le dichromate de bismuthyle $(BiO)_2Cr_2O_7$ ; le chromate acide de bismuthyle $H(BiO)CrO_4$ ; le chromate double de bismuthyle et de potassium $K(BiO)Cr_3O_{10}$ ; le molybdate de bismuth $Bi_2(MoO_4)_3$ ; le tungstate de bismuth $Bi_2(WO_4)_3$ ; le molybdate double de bismuth et de sodium $NaBi(MoO_4)_2$ ; le permanganate basique de bismuth $Bi_2O_2(OH)MnO_4$, l'oxyde de bismuth et de germanium $Bi_{12}GeO_{20}$,
- comme sels d'acides organiques aliphatiques ou aromatiques : l'acétate de bismuth $Bi(C_2H_3O_2)_3$ ; le propionate de bismuthyle $(BiO)C_3H_5O_2$ ; le benzoate basique de bismuth $C_6H_5CO_2Bi(OH)_2$ ; le salicylate de bismuthyle $C_6H_4CO_2(BiO)(OH)$ ; l'oxalate de bismuth $(C_2O_4)_3Bi_2$ ; le tartrate de bismuth $Bi_2(C_4H_4O_6)_3$, $6H_2O$ ; le lactate de bismuth $(C_6H_9O_5)OBi$, $7H_2O$ ; le citrate de bismuth $C_6H_5O_7Bi$,
- comme phénates: le gallate basique de bismuth $C_7H_7O_7Bi$ ; le pyrogallate basique de bismuth $C_6H_3(OH)_2(OBi)(OH)$.

Comme autres composés minéraux ou organiques conviennent également: le phosphure de bismuth BiP ; l'arséniure de bismuth $Bi_3As_4$ ; le bismuthate de sodium $NaBiO_3$ ; les acides bismuth-thiocyaniques $H_2[Bi(CNS)_5]$, $H_3[Bi(CNS)_6]$ et leurs sels de sodium et potassium ; la triméthylbismuthine $Bi(CH_3)_3$, la triphénylbismuthine $Bi(C_6H_5)_3$.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe de catalyseurs qui conviennent particulièrement bien à la réalisation de l'invention est constitué par : les oxydes de bismuth $Bi_2O_3$ et $Bi_2O_4$, l'hydroxyde de bismuth $Bi(OH)_3$ ; le sulfate neutre de bismuth $Bi_2(SO_4)_3$; le chlorure de bismuth $BiCl_3$ ; le bromure de bismuth $BiBr_3$; l'iodure de bismuth $BiI_3$ ; le nitrate neutre de bismuth $Bi(NO_3)_3$, $5H_2O$ ; le nitrate de bismuthyle $BiO(NO_3)$ ; le carbonate de bismuthyle $(BiO)_2CO_3$, $O,5H_2O$ ; l'acétate de bismuth $Bi(C_2H_3O_2)_3$ ; le salicylate de bismuthyle $C_6H_4CO_2(BiO)(OH)$.

En ce qui concerne la source d'halogène, on peut faire appel à tout composé susceptible d'apporter des ions halogène permettant de générer in situ l'halogénure de bismuth.

Comme exemples de sources d'halogène, on peut faire appel à l'halogène sous forme moléculaire ; à tout halogénure d'acide minéral ou organique, et plus particulièrement des acides carboxyliques aliphatiques à tout sel métallique ou métalloïdique, minéral ou organique susceptible de générer une forme halogénée du bismuth.

Comme exemples plus spécifiques, on peut mentionner entre autres, le chlore ou le brome : l'acide chlorhydrique, l'acide bromhydrique ; le chlorure d'acétyle ; le chlorure de silicium $SiCl_4$, les halogénosilanes tels que $Me_3SiCl$, $Me_2SiCl_2$,, $MeSiCl_3$, $PhMe_2SiCl$, le chlorure de phosphore $PCl_3$, le chlorure de soufre $SCl_2$.

Une variante préférée du procédé de l'invention consiste à conduire la réaction dans un solvant organique.

On choisit de préférence un solvant du substrat de départ.

Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques.

A titre d'exemples d'hydrocarbures aliphatiques, on peut citer plus particulièrement les paraffines tels que notamment, l'hexane, l'heptane, l'octane, le nonane, le décane, le undécane, le dodécane, le tétradécane ou le cyclohexane, et le naphtalène et les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachlorométhane, le tétrachloroéthylène, l'hexachloroéthane ; les hydrocarbures partiellement chlorés tels que le dichlorométhane, le chloroforme, le 1,2-dichloroéthane, le 1,1,1 -trichloroéthane, le 1,1 ,2,2-tétrachloroéthane, le pentachloroéthane, le trichloroéthylène, le 1-chlorobutane, le 1,2-dichlorobutane ; le monochlorobenzène, le 1,2-dichlorobenzène, le 1 ,3-dichlorobenzène, le 1,4-dichlorobenzène , le 1,2,4-trichlorobenzène ou des mélanges de différents chlorobenzènes; le bromoforme, le bromoéthane ou le 1,2-dibromoéthane ; le monobromobenzène ou des mélanges de monobromobenzène avec un ou plusieurs dibromobenzènes; le 1-bromonaphtalène.

On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1 ,2-diméthoxyéthane), le diméthyléther du diéthylèneglycol (ou 1,5-diméthoxy 3-oxapentane) ; l'oxyde de biphényle ou de benzyle; le dioxane, le tétrahydrofuranne (THF).

Les solvants préférés sont : le dichlorométhane, le tétrachlorométhane, le THF et l'oxyde de diéthyle.

On peut également utiliser un mélange de solvants organiques.

On utilise préférentiellement, le substrat de départ comme solvant réactionnel.

Il est souhaitable que le solvant soit anhydre.

Dans une première étape du procédé de l'invention, on effectue l'acylation du composé aromatique. Dans une étape suivante, on conduit l'hydrolyse de la masse réactionnelle obtenue.

Le rapport entre le nombre de moles de composé aromatique et le nombre de moles d'agent d'acylation peut varier car le substrat peut servir de solvant réactionnel. Ainsi, le rapport peut aller de 0,1 à 10, de préférence aux environs de 4,0.

La quantité de catalyseur mis en oeuvre est déterminée de telle sorte que le rapport entre le nombre de moles de catalyseur et le nombre de moles d'agent d'acylation est inférieur à 1,0 et varie, de préférence, entre 0,001 et 0,8, et encore plus préférentiellement entre 0,02 et 0,2.

Pour ce qui est de la quantité de solvant organique mis en oeuvre, elle est choisie généralement de telle sorte que le rapport entre le nombre de moles de solvant organique et le nombre de moles de composé aromatique varie, de préférence, entre 0 et 100 et encore plus préférentiellement entre 0 et 50.

La température à laquelle est mise en oeuvre la réaction d'acylation dépend de la réactivité du substrat de départ

et de celle de l'agent d'acylation.

Elle se situe entre 20°C et 200°C, de préférence entre 40°C et 150°C.

Généralement, la réaction est conduite à pression atmosphérique mais des pressions plus faibles ou plus élevées peuvent également convenir.

D'un point de vue pratique, il n'y a pas de contraintes au niveau de la mise en oeuvre des réactifs. Ils peuvent être introduits dans un ordre quelconque.

Après mise en contact des réactifs, on porte le mélange réactionnel à la température souhaitée.

Une autre variante de l'invention consiste à chauffer l'un des réactifs (agent d'acylation ou composé aromatique) avec le catalyseur, puis à introduire l'autre réactif.

La durée de la réaction est fonction de nombreux paramètres. Le plus souvent, elle est de 30 minutes à 8 heures.

Dans une étape suivante du procédé de l'invention, on effectue un traitement d'hydrolyse de la masse réactionnelle obtenue.

La quantité d'eau mise en oeuvre peut varier très largement. Le rapport entre le nombre de moles d'eau et le nombre de moles de composé aromatique peut varier, entre 10 et 100, et de préférence entre 20 et 30.

A cet effet, un mode préféré de réalisation de cette opération consiste à ajouter la masse réactionnelle sur un pied d'eau portée à une température comprise entre 0°C et 100°C, de préférence entre 15°C et 30°C.

Une variante de l'invention consiste à remplacer l'eau par une solution basique, généralement de soude, carbonate ou hydrogénocarbonate de sodium ayant une concentration de 5 à 20 % en poids.

On sépare le catalyseur, de préférence par filtration. Le catalyseur peut être recyclé après séchage.

En fin de réaction, on récupère le produit désiré à savoir l'alkylcétone alkoxyaromatique en phase organique.

On sépare les phases aqueuse et organique.

On lave une ou plusieurs fois, la phase organique, de préférence 2 fois, avec l'eau.

On sépare les phases aqueuse et organique.

On récupère ensuite l'alkylcétone alkoxyaromatique à partir de la phase organique selon les techniques connues, par élimination du solvant organique par distillation ou par cristallisation.

Le procédé de l'invention est particulièrement bien adapté à la préparation de la 4-méthoxyacétophénone et de la 3,4-diméthoxyacétophénone dénommée couramment acétovératrole, par acétylation respectivement de l'anisole ou du vératrole.

L'intérêt du procédé de l'invention est qu'il permet de faire appel à une quantité catalytique de catalyseur au bismuth.

Un avantage du procédé de l'invention est que la réaction d'acylation s'effectue sans qu'il y ait une O-déméthylation du vératrole.

De plus, on note la présence de peu d'isomère ortho en raison du caractère para-orienteur du catalyseur.

Les exemples qui suivent, illustrent l'invention sans toutefois la limiter.

Dans les exemples, les rendements mentionnés correspondent à la définition suivante:

$$\text{Rendement : } RR_{E.A.} = \frac{\text{nombre de moles d'éther aromatique introduites}}{\text{nombre de moles d'alkylcétone alkoxy aromatique formées}}\ \%$$

$$\text{Rendement : } RR_{A.A.} = \frac{\text{nombre de moles d'agent d'acylation introduites}}{\text{nombre de moles d'alkylcétone alkoxy aromatique formées}}\ \%$$

Exemples 1 à 10

Dans un réacteur de 25 ml équipé d'un réfrigérant, d'un thermomètre et muni d'un agitateur magnétique, on charge :

- 10,8 g d'anisole,
- 9,42 g de chlorure d'acétyle,
- 10 ml de solvant dont la nature est précisée dans le tableau suivant,
- x % molaire exprimé par rapport à l'anisole d'un catalyseur dont la nature est également précisée dans le tableau suivant.

Le mélange réactionnel est chauffé à reflux selon une durée mentionnée dans le tableau.

En fin de réaction, une partie de la masse réactionnelle est prélevée, hydrolysée par une solution de carbonate de sodium à 10 %.

La phase organique est séparée de la phase aqueuse, le solvant est évaporé puis les produits sont analysés par RMN.

Les rendements en 4-méthoxyacétophénone sont exprimés par rapport à l'anisole engagé.

Tableau I

| N° ex | Nature du catalyseur | % molaire du catalyseur par rapport à l'anisole | Nature du solvant (rapport volumique) | Durée (h) | Rendement $RR_{E.A.}$ (%) |
|---|---|---|---|---|---|
| 1 | $BiCl_3$ | 5 | Dichlorométhane Ether éthylique (10/1) | 3 | 28 |
| 2 | $BiCl_3$ | 15 | Dichlorométhane Ether éthylique (10/1) | 3 | 55 |
| 3 | $BiCl_3$ | 5 | Dichlorométhane | 8 | 40 |
| 4 | $BiCl_3$ | 5 | Dichlorométhane Ether éthylique (10/1) | 8 | 33 |
| 5 | $BiCl_3$ | 15 | Dichlorométhane Ether éthylique (10/1) | 4 | 47 |
| 6 | $BiCl_3$ | 5 | Tétrachlorométhane | 4 | 18 |
| 7 | $BiCl_3$ | 5 | Dichlorométhane THF (10/1) | 3 | 19 |
| 8 | BiOCl | 5 | Dichlorométhane Ether éthylique (10/1) | 4 | 23 |
| 9 | $Bi_2O_3$ | 5 | Dichlorométhane Ether éthylique (10/1) | 3 | 55 |
| 10 | $Bi_2O_3$ | 10 | Dichlorométhane Ether éthylique (10/1) | 2 | 60 |

Exemple 11

On reproduit le mode opératoire donné pour les exemples 1 à 10, à la différence près que l'on conduit la réaction à la température ambiante.

Les résultats obtenus sont les suivants:

Tableau II

| N° ex | Nature du catalyseur | % molaire du catalyseur par rapport à l'anisole | Nature du solvant (rapport volumique) | Durée (h) | Rendement $RR_{E.A.}$ (%) |
|---|---|---|---|---|---|
| 11 | $BiCl_3$ | 5 | Dichlorométhane Ether éthylique (10/1) | 120 | 5 |

Exemples 12 à 16

On opère selon le mode opératoire des exemples 1 à 10, sauf que l'on change la nature de l'agent d'acylation.

Tableau III

| N° ex | Nature du catalyseur | % molaire du catalyseur par rapport à l'anisole | Nature du solvant (rapport volumique) | Nature de l'agent d'acylation | Durée (h) | Rendement $RR_{E.A.}$ (%) |
|---|---|---|---|---|---|---|
| 12 | $BiCl_3$ | 10 | Benzène | Chlorure d'isobutanoyle | 3 | 62 |

Suite du Tableau sur la page suivante

Tableau III (suite)

| N° ex | Nature du catalyseur | % molaire du catalyseur par rapport à l'anisole | Nature du solvant (rapport volumique) | Nature de l'agent d'acylation | Durée (h) | Rendement $RR_{E.A.}$ (%) |
|---|---|---|---|---|---|---|
| 13 | $Bi_2O_3$ | 5 | Dichlorométhane Ether éthylique (10/1) | Chlorure d'isobutanoyle | 5 | 69 |
| 14 | $Bi_2O_3$ | 5 | Benzène | Chlorure d'isobutanoyle | 4 | 68 |
| 15 | $Bi_2O_3$ | 5 | Dichlorométhane Ether éthylique (10/1) | Chorure de pivaloyle | 4 | 70 |
| 16 | $BiCl_3$ | 10 | Dichlorométhane Ether éthylique (10/1) | Anhydride acétique | 4 | 8 |

Exemples 17 à 19

Dans un réacteur de 25 ml équipé d'un réfrigérant, d'un thermomètre et muni d'un agitateur magnétique, on charge :

- 1,08 g (0,01 mol) d'anisole,
- 2,2 g (0,028 mol) de chlorure d'acétyle,
- x % molaire exprimé par rapport à l'anisole d'un catalyseur dont la nature est également précisée dans le tableau suivant.

Le mélange réactionnel est chauffé à reflux selon une durée mentionnée dans le tableau.
En fin de réaction, on ajoute 30 ml d'une solution aqueuse de soude à 5 %.
Après traitement selon l'exemple 1, les produits organiques sont analysés par RMN.
Les rendements en 4-méthoxyacétophénone sont exprimés par rapport à l'anisole engagé.

Tableau IV

| N° ex | Nature du catalyseur | % molaire du catalyseur par rapport à l'anisole | Nature de l'agent d'acylation | Durée (mn) | Rendement $RR_{E.A.}$ (%) |
|---|---|---|---|---|---|
| 17 | $BiCl_3$ | 5 | Chlorure d'acétyle | 45 | 43 |
| 18 | $BiCl_3$ | 15 | Chlorure d'acétyle | 30 | 37 |
| 19 | $Bi_2O_3$ | 5 | Chlorure d'acétyle | 90 | 69 |

Exemples 20 à 31

Dans les exemples suivants, on opère en présence d'un excès du substrat aromatique.
Dans certains exemples, on change la nature du substrat aromatique et de l'agent d'acylation.
Dans un réacteur de 25 ml équipé d'un réfrigérant, d'un thermomètre et muni d'un agitateur magnétique, on charge :

- 40 mmol d'un substrat aromatique,
- 10 mmol de l'agent d'acylation,
- x % molaire exprimé par rapport à l'agent d'acylation d'un catalyseur dont la nature est également précisée dans le tableau suivant.

Le mélange réactionnel est chauffé à la température et selon une durée mentionnées dans le tableau.
En fin de réaction, on ajoute 20 ml d'une solution aqueuse de soude à 5 %.
Après traitement selon l'exemple 1, les produits organiques sont analysés par RMN.
Les rendements en cétones aromatiques sont exprimés par rapport à l'agent d'acylation engagé.

Tableau V

| N° ex | Nature du substrat aromatique | Nature du catalyseur | % molaire du catalyseur par rapport à l'agent d'acylation | Nature de l'agent d'acylation | Température (°C) | Durée (h) | Rendement $RR_{A.A.}$ (%) |
|---|---|---|---|---|---|---|---|
| 20 | Anisole | $BiCl_3$ | 10 | Chlorure d'acétyle | 50 | 1 | 60 |
| 21 | Anisole | $BiCl_3$ | 5 | Chlorue d'isobutanoyle | 70 | 2 | 80 |
| 22 | Anisole | $BiCl_3$ | 10 | Chlorure de pivaloyle | reflux | 3 | 95 |
| 23 | Anisole | $BiCl_3$ | 10 | Chlorure de benzoyle | 85 | 6 | 100 |
| 24 | Vératrole | $BiCl_3$ | 10 | Chlorure d'acétyle | 50 | 1 | 80 |
| 25 | Vératrole | $BiCl_3$ | 10 | Chlorure d'isobutanoyle | 70 | 2 | 82 |
| 26 | Anisole | $BiCl_3$ | 10 | Anhydride acétique | reflux | 6,5 | 87 |
| 27 | Anisole | $BiCl_3$ | 10 | Anhydride isobutyrique | reflux | 6 | 67 |
| 28 | Anisole | $BiCl_3$ | 10 | Anhydride acétique | reflux | 3,5 | 100 |
| 29 | Anisole | $BiCl_3$ | 10 | Anhydride isobutyrique | reflux | 6 | 65 |
| 30 | Vératrole | $BiCl_3$ | 10 | Anhydride acétique | reflux | 7,5 | 65 |
| 31 | Vératrole | $BiCl_3$ | 10 | Anhydride isobutyrique | reflux | 7 | 24 |

Exemple 32

Dans un réacteur de 100 ml équipé d'un réfrigérant, d'un thermomètre et muni d'un agitateur magnétique, on charge :

- 43,2 g (0,4 mol) d'anisole,
- 10,6 g (10 mmol) du chlorure d'isobutanoyle,
- 3,15 g (1 mmol) de chlorure de bismuth.

Le mélange réactionnel est chauffé à 85°C à l'aide d'un bain de paraffine, pendant 6 heures.

En fin de réaction, après refroidissement, on hydrolyse le milieu réactionnel par ajout de 100 ml d'une solution aqueuse de soude 1,25 N.

Le catalyseur est éliminé par filtration après décantation ; la phase aqueuse étant extraite par 100 ml d'éther.

L'ensemble des phases organiques sont séchées sur sulfate de sodium, filtrées et concentrées.

3 g de la phase organique sont prélevés et analysés par chromatographie sur colonne de Silicagel Merck 60. L'élution au pentane permet d'extraire l'anisole ; la cétone attendue étant éluée à l'éther éthylique.

Le rendement en 4-méthoxyisobutyrophénone est de 87 % par rapport au chlorure d'isobutanoyle.

Exemples 33 à 44

Dans ces exemples, on met en évidence l'influence de la nature et de la quantité de catalyseur engagé.

Les conditions de l'exemple précédent sont reproduites.

Les durées de réaction qui sont différentes, sont précisées dans le tableau suivant.

Le substrat aromatique est l'anisole dans tous les exemples. L'anisole utilisé en excès sert de solvant réactionnel.

Tableau VI

| N° ex | Nature du catalyseur | % molaire du catalyseur par rapport à l'agent d'acylation | Durée (h) | Rendement $RR_{A.A.}$ (%) |
|---|---|---|---|---|
| 32 | $BiCl_3$ | 10 | 6 | 87 |
| 33 | $BiCl_3$ | 1 | 6,5 | 40 |
| 34 | $Bi_2O_3$ | 5 | 4 | 74 |
| 35 | $Bi_2O_3$ | 0,5 | 4 | 35 |
| 36 | $Bi_{12}GeO_{20}$ | 1 | 2 | 52 |
| 37 | $2Bi_2O_3$, $3ZnO_2$ | 2,5 | 3,5 | 53 |
| 38 | $(CH_3COO)_3Bi$ | 10 | 3 | 49 |
| 39 | $(BIO)_2CO_3$ | 10 | 6 | 48 |
| 40 | $BiI_3$ | 10 | 6 | 11,5 |
| 41 | $BIBr_3$ | 10 | 6 | 54 |
| 42 | $C_6H_4CO_2(BiO)(OH)$ | 10 | 6,5 | 51 |
| 43 | BiOCl | 10 | 6 | 45 |
| 44 | $Ph_3Bi$ | 10 | 6,5 | 62 |

Exemple 45

Dans un réacteur de 100 ml équipé d'un réfrigérant, d'un thermomètre et muni d'un agitateur magnétique, on charge :

- 55,2 g (0,4 mol) de vératrole,
- 10,6 g (10 mmol) du chlorure d'isobutanoyle,

- 3,15 g (1 mmol) de chlorure de bismuth.

Le mélange réactionnel est chauffé à 85°C à l'aide d'un bain de paraffine, pendant 6 heures.

En fin de réaction, après refroidissement, on hydrolyse le milieu réactionnel par ajout de 100 ml d'une solution aqueuse de soude 1,25 N.

Le catalyseur est éliminé par filtration après décantation ; la phase aqueuse étant extraite par 100 ml d'éther.

L'ensemble des phases organiques sont séchées sur sulfate de sodium, filtrées et concentrées.

3 g de la phase organique sont distillés. On recueille à 118°C sous 0,1 mm de mercure, la 3,4-diméthoxyisobutyrophénone.

Le rendement en 3,4-diméthoxyisobutyrophénone est de 88 % par rapport au chlorure d'isobutanoyle.

Exemple 46

Dans ces exemples, on met en évidence l'influence de la nature et de la quantité de catalyseur engagé.

Les conditions de l'exemple précédent sont reproduites.

Les durées de réaction qui sont différentes sont précisées dans le tableau suivant.

Le substrat aromatique est le vératrole dans tous les exemples. Le vératrole utilisé en excès sert de solvant réactionnel.

Tableau VII

| N°ex | Nature du catalyseur | % molaire du catalyseur par rapport à l'agent d'acylation | Durée (h) | Rendement $RR_{A.A.}$ (%) |
|---|---|---|---|---|
| 45 | $BiCl_3$ | 10 | 6,5 | 88 |
| 46 | $Bi_2O_3$ | 5 | 4 | 58 |

Exemples comparatifs 47 à 52

Dans ces exemples, on change la nature du catalyseur par d'autres catalyseurs métalliques.

Les conditions de l'exemple 32 précédent sont reproduites.

Le substrat aromatique est l'anisole dans tous les exemples. L'anisole utilisé en excès sert de solvant réactionnel.

Tableau VIII

| N°ex | Nature du catalyseur | % molaire du catalyseur par rapport à l'agent d'acylation | Durée (h) | Rendement $RR_{A.A.}$ (%) |
|---|---|---|---|---|
| 32 | $BiCl_3$ | 10 | 6 | 87 |
| 47 | $SbCl_3$ | 10 | 6 | 27 |
| 48 | $ZnCl_2$ | 5 | 6 | 46 |
| 49 | $FeCl_3$ | 5 | 6 | 58 |
| 50 | $Sb_2O_3$ | 5 | 4 | 20 |
| 51 | $Fe_2O_3$ | 5 | 6,5 | 5 |
| 52 | ZnO | 5 | 6,5 | 60 |

Exemple 53

Dans un réacteur de 100 ml équipé d'un réfrigérant, d'un thermomètre et muni d'un agitateur magnétique, on charge :

- 43,2 g (0,4 mol) d'anisole,
- 10,6 g (10 mmol) du chlorure d'hexanoyle,
- 3,15 g (1 mmol) de chlorure de bismuth.

Le mélange réactionnel est chauffé à 85°C à l'aide d'un bain de paraffine, pendant 6 heures.

Toutes les deux heures, on introduit 10,6 g (10 mmol) du chlorure d'hexanoyle.

Après 6 heures de chauffage ce qui correspond à une introduction totale de 40 mmol de chlorure d'hexanoyle, le mélange réactionnel est refroidi.

On hydrolyse le milieu réactionnel par ajout de 100 ml d'une solution aqueuse de soude 1,25 N.

Le catalyseur est éliminé par filtration après décantation; la phase aqueuse étant extraite par 100 ml d'éther.

L'ensemble des phases organiques sont séchées sur sulfate de sodium, filtrées et concentrées.

3 g de la phase organique sont prélevés et analysés par chromatographie sur colonne de Silicagel Merck 60. L'élution au pentane permet d'extraire l'anisole ; la cétone attendue étant éluée à l'éther éthylique.

Le rendement en phénylpentylcétone est de 55 % par rapport au chlorure d'hexanoyle.

Exemple 54

Dans cet exemple, on opère comme dans l'exemple 34, le catalyseur récupéré par filtration lors du traitement de l'exemple 34 est recyclé et donc réengagé dans les mêmes conditions que décrites dans l'exemple 34.

Le catalyseur introduit à une teneur de 74 % en bismuth ce qui représente compte tenu des masses engagées, 96 % du bismuth engagé dans l'exemple 34.

On obtient un rendement en 4-méthoxyisobutyrophénone de 47 % ce qui montre que le catalyseur est toujours actif.

Exemple 55

Dans cet exemple, on met en évidence le fait que le catalyseur ne dégrade pas le produit obtenu.

Dans un réacteur de 50 ml, on charge :

- 5 g de 4-méthoxyacétophénone,
- 2,1 g de trichlorure de bismuth.

Le mélange réactionnel est chauffé à 150°C, pendant 2 heures.

Après refroidissement, la masse réactionnelle est hydrolysée par 100 ml d'une solution aqueuse de soude 1,25 N.

La phase aqueuse est extraite à l'éther.

La phase organique est séchée sur sulfate de sodium, filtrée et concentrée.

Le spectre RMN montre qu'il n'y a pas de dégradation de la 4-méthoxyacétophénone.

Exemples 56 à 60

Dans ces exemples, on met en évidence le rôle des halogénures dans le milieu.

Dans un réacteur de 25 ml équipé d'un réfrigérant, d'un thermomètre et muni d'un agitateur magnétique, on charge :

- 1,08 g (0,01 mol) d'anisole,
- 2,55 g (0,025 mol) d'anhydride acétique,
- x % molaire exprimé par rapport à l'agent d'acylation d'un catalyseur dont la nature est précisée dans le tableau suivant.

Le mélange réactionnel est chauffé à 80°C à l'aide d'un bain de paraffine, pendant une durée également mentionnée dans ledit tableau.

En fin de réaction, on ajoute 20 ml d'une solution aqueuse de soude à 5 %; le catalyseur est éliminé par filtration après décantation ; la phase aqueuse étant extraite par 20 ml d'éther.

La phase organique est séchée sur sulfate de sodium, filtrée et concentrée.

Le produit est analysé par RMN.

Le rendement en 4-méthoxyacétophénone est exprimé par rapport à l'agent d'acylation.

Tableau IX

| N° ex | Nature du catalyseur | % molaire du catalyseur par rapport à l'agent d'acylation | Durée (h) | Rendement $RR_{A.A.}$ (%) |
|---|---|---|---|---|
| 56 | $Bi_2O_3$ | 10 | 4 | 0 |
| 57 | $C_6H_4CO_2(BiO)(OH)$ | 10 | 4,5 | 0 |
| 58 | $Bi_2O_3$, $6CH_3COCl$ | 5 | 7 | 79 |
| 59 | $Bi_2O_3$, $6Me_3SiCl$ | 5 | 7 | 39 |
| 60 | $BiCl_3$ | 10 | 6 | 100 |

Exemple 61

Dans ces exemples, on compare l'influence de la nature de l'halogénure d'acide.
On reproduit le mode opératoire donné dans l'exemple 41.
Les résultats obtenus sont les suivants:

Tableau X

| N° ex | Nature du catalyseur | Nature de l'agent d'acylation | % molaire du catalyseur par rapport à l'agent d'acylation | Durée (h) | Rendement $RR_{A.A.}$ (%) |
|---|---|---|---|---|---|
| 41 | $BiBr_3$ | chlorure d'isobutanoyle | 10 | 6 | 54 |
| 61 | $BiBr_3$ | bromure d'acétyle | 10 | 4 | 21 |

**Revendications**

**1 -** Procédé d'acylation d'un composé aromatique porteur d'au moins un groupe hydroxyle et/ou alkoxy qui consiste à faire réagir ledit composé aromatique avec un agent d'acylation, en présence d'un catalyseur puis à récupérer le produit formé caractérisé par le fait que l'on conduit la réaction d'acylation en présence d'une quantité efficace d'un halogénure de bismuth ou tout composé susceptible de le générer in situ.

**2 -** Procédé selon la revendication 1 caractérisé par le fait que le composé aromatique répond à la formule générale (I) :

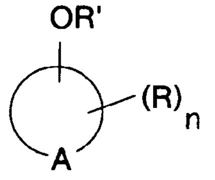

(I)

dans laquelle :

- A symbolise le reste d'un cycle formant tout ou partie d'un système carbocyclique aromatique, monocyclique ou polycyclique, système comprenant au moins un groupe OR': ledit reste cyclique pouvant porter un ou plusieurs substituants,

- R représente un ou plusieurs substituants, identiques ou différents,

- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,

- n est un nombre inférieur ou égal à 4.

**3 -** Procédé selon l'une des revendications 1 et 2 caractérisé par le fait que le composé aromatique répond à la formule générale (I) dans laquelle R' représente:

- un atome d'hydrogène,
- un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
- un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
- un radical carbocyclique saturé ou comprenant 1 ou 2 insaturations dans le cycle, ayant généralement de 3 à 7 atomes de carbone, de préférence, 6 atomes de carbone dans le cycle; ledit cycle pouvant être substitué.
- un radical carbocyclique aromatique, de préférence monocyclique ayant généralement au moins 4 atomes de carbone, de préférence, 6 atomes de carbone dans le cycle; ledit cycle pouvant être substitué.

**4 -** Procédé selon la revendication 1 caractérisé par le fait que le composé aromatique répond à la formule générale (I) dans laquelle R' représente un radical alkyle linéaire ou ramifié ayant de 1 à 4 atomes de carbone, de préférence un radical méthyle ou un radical phényle.

**5 -** Procédé selon l'une des revendications 1 à 4 caractérisé par le fait que le composé aromatique répond à la formule générale (I) dans laquelle le reste A représente le reste d'un composé carbocyclique aromatique, monocyclique ayant au moins 4 atomes de carbone et de préférence 6 atomes de carbone ou le reste d'un composé carbocyclique polycyclique : le reste A pouvant porter un ou plusieurs substituants sur le noyau aromatique.

**6 -** Procédé selon l'une des revendications 1 à 5 caractérisé par le fait que le composé aromatique répond à la formule (Ia) :

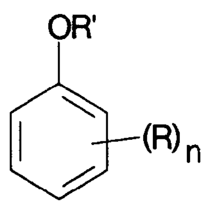

(Ia)

dans laquelle :

- n est un nombre inférieur ou égal à 4, de préférence égal à 0, 1 ou 2,
- le radical R' représente un atome d'hydrogène ou un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou phényle,
- le ou les radicaux R représentent l'un des atomes ou groupes suivants:
  - . un atome d'hydrogène,
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,

. un groupe hydroxyle,

. un atome d'halogène, de préférence un atome de fluor, chlore ou brome.

- les radicaux R' et R et les 2 atomes successifs du cycle benzénique peuvent former entre eux, un cycle ayant de 5 à 7 atomes, comprenant éventuellement un autre hétéroatome.

**7 -** Procédé selon l'une des revendications 1 à 6 caractérisé par le fait que le composé aromatique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence l'oxygène: les radicaux R' et R formant de préférence un radical méthylène dioxy ou éthylène dioxy.

**8 -** Procédé selon l'une des revendications 1 à 7 caractérisé par le fait que le composé aromatique répond à la formule (Ia) dans laquelle n est égal à 1, les radicaux R et R' représentant tous deux, des radicaux alkoxy, identiques ou différents.

**9 -** Procédé selon la revendication 1 et 2 caractérisé par le fait que le composé aromatique est le phénol, l'anisole ou le vératrole.

**10 -** Procédé selon l'une des revendications 1 à 9 caractérisé par le fait que l'agent d'acylation répond à la formule (II) :

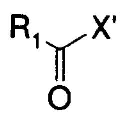

**(II)**

dans laquelle:

- $R_1$ représente :
  . un radical aliphatique saturé ou insaturé, linéaire ou ramifié ayant de 1 à 24 atomes de carbone ; un radical cycloaliphatique, saturé, insaturé ou aromatique, monocyclique ou polycyclique, ayant de 4 à 8 atomes de carbone ; un radical aliphatique saturé ou insaturé, linéaire ou ramifié, porteur d'un substituant cyclique,

- X' représente :

. un atome d'halogène, de préférence un atome de chlore ou de brome,

. un radical -O-CO-$R_2$ avec $R_2$, identique ou différent de $R_1$, ayant la même signification que $R_1$.

**11 -** Procédé selon la revendication 10 caractérisé par le fait que l'agent d'acylation répond à la formule (II) dans laquelle X' représente un atome de chlore et $R_1$ représente un radical alkyle linéaire ou ramifié ayant de 1 à 12 atomes de carbone : la chaîne hydrocarbonée pouvant être éventuellement interrompue par un hétéroatome ou par un groupe fonctionnel ou porteuse d'un substituant; $R_1$ représente un radical phényle éventuellement substitué ; ou X' représente un radical -O-CO-$R_2$. dans laquelle $R_1$ et $R_2$ sont identiques, représentent un radical alkyle ayant de 1 à 4 atomes de carbone.

**12 -** Procédé selon la revendication 10 et 11 caractérisé par le fait que l'agent d'acylation est choisi parmi:

- le chlorure d'acétyle,

- le chlorure de propanoyle,

- le chlorure d'isobutanoyle,

- le chlorure de pivaloyle,
- l'anhydride acétique,
- l'anhydride isobutyrique,
- l'anhydride trifluoroacétique.

**13 -** Procédé selon l'une des revendications 1 à 12 caractérisé par le fait que le catalyseur est un halogénure de bismuth de préférence, chlorure, bromure, iodure et plus préférentiellement le chlorure de bismuth $BiCl_3$ ; le bromure de bismuth $BiBr_3$ ; l'iodure de bismuth $BiI_3$ ou tout composé du bismuth associé à une source d'halogène.

**14 -** Procédé selon la revendication 13 caractérisé par le fait que le composé du bismuth est choisi parmi un dérivé organique ou inorganique du bismuth pris dans le groupe formé par: les oxydes de bismuth ; les hydroxydes de bismuth ; les sels de bismuth ou de bismuthyle d'hydracides minéraux, de préférence les sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels d'oxyacides dérivés de métaux de transition : de préférence l'oxyde de bismuth et de germanium $Bi_{12}GeO_{20}$ ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

**15 -** Procédé selon la revendication 13 caractérisé par le fait que la source d'halogène est : l'halogène sous forme moléculaire ; tout halogénure d'acide minéral ou organique, et plus particulièrement des acides carboxyliques aliphatiques ; tout sel métallique ou métalloïdique, minéral ou organique susceptible de générer une forme halogénée du bismuth.

**16 -** Procédé selon la revendication 15 caractérisé par le fait que la source d'halogène est choisie parmi : le chlore ou le brome : l'acide chlorhydrique, l'acide bromhydrique ; le chlorure d'acétyle ; le chlorure de silicium $SiCl_4$, les halogénosilanes tels que $Me_3SiCl$, $Me_2SiCl_2$, $MeSiCl_3$, $PhMe_2SiCl$, le chlorure de phosphore $PCl_3$, le chlorure de soufre $SCl_2$.

**17 -** Procédé selon l'une des revendications 1 à 16 caractérisé par le fait que le solvant réactionnel est le substrat aromatique ou un solvant organique aprotique, apolaire et peu basique.

**18 -** Procédé selon la revendication 17 caractérisé par le fait que le solvant organique est choisi parmi les hydrocarbures aliphatiques et/ou aromatiques éventuellement halogénés, de préférence chlorés, les éther-oxydes aliphatiques, cycloaliphatiques ou aromatiques.

**19 -** Procédé selon la revendication 18 caractérisé par le fait que le solvant organique est le dichlorométhane, le tétrachlorométhane, le THF et l'oxyde de diéthyle.

**20 -** Procédé selon l'une des revendications 1 à 19 caractérisé par le fait que le rapport entre le nombre de moles de composé aromatique et le nombre de moles d'agent d'acylation varie entre 0,1 et 10, de préférence aux environs de 4.

**21 -** Procédé selon l'une des revendications 1 à 20 caractérisé par le fait que la quantité de catalyseur mis en oeuvre est telle que le rapport entre le nombre de moles de catalyseur et le nombre de moles d'agent d'acylation est inférieur à 1,0 et varie, de préférence, entre 0,001 et 0,8, et encore plus préférentiellement entre 0,02 et 0,2.

**22 -** Procédé selon l'une des revendications 1 à 21 caractérisé par le fait que la température à laquelle est mise en oeuvre la réaction d'acylation se situe entre 20°C et 200°C, de préférence entre 40°C et 150°C.

Office européen des brevets

# RAPPORT DE RECHERCHE EUROPEENNE

Numero de la demande
EP 95 40 1929

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | EP-A-0 067 698 (SAGAMI CHEMICAL RESEARCH CENTER)<br>* le document en entier *<br>--- | 1 | C07C45/46<br>C07C49/84<br>C07C49/825 |
| X | EP-A-0 600 318 (HOECHST AKTIENGESELLSCHAFT)<br>* le document en entier *<br>----- | 1 | |
| | | | **DOMAINES TECHNIQUES RECHERCHES (Int.Cl.6)** |
| | | | C07C |

Le présent rapport a été établi pour toutes les revendications

| Lieu de la recherche | Date d'achèvement de la recherche | Examinateur |
|---|---|---|
| LA HAYE | 30 Novembre 1995 | Bonnevalle, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)